# EUROPÄISCHE PATENTSCHRIFT

(11) **EP 3 562 442 B1**
(45) Veröffentlichungstag und Bekanntmachung des Hinweises auf die Patenterteilung: **27.01.2021**
(21) Anmeldenummer: 17821856.6
(22) Anmeldetag: 12.12.2017
(51) Int. Cl.: A61F 2/54, A61F 2/58, B25J 15/00, A61F 2/76

(54) **BEFESTIGUNGSANORDNUNG EINER PROTHESENHAND AN EINEM UNTERARMSCHAFT UND PROTHESENSYSTEM**
ASSEMBLY DEVICE OF A PROSTHESIS HAND TO A LOWER ARM AND PROSTHESIS SYSTEM
AGENCEMENT D'ASSEMBLAGE D'UNE PROTHÈSE DE MAIN SUR UN SYSTÈME DE SOUS-BAS ET DE PROTHÈSE

(30) Priorität: 27.12.2016 DE 102016125724
(43) Veröffentlichungstag der Anmeldung: 06.11.2019
(73) Patentinhaber: Otto Bock Healthcare Products GmbH, 1110 Wien (AT)
(72) Erfinder: FITZ, Stefan, 1030 Wien (AT); WEISSMAYER, Klaus, 1210 Wien (AT)
(74) Vertreter: Gramm, Lins & Partner Patent- und Rechtsanwälte PartGmbB
(86) Internationale Anmeldenummer: PCT/EP2017/082440
(87) Internationale Veröffentlichungsnummer: WO 2018/121983

(56) Entgegenhaltungen:
- WO-A1-2016/051138
- GB-A- 2 498 788
- US-A1- 2011 136 376
- US-A1- 2011 257 765
- US-A1- 2014 114 439
- US-A1- 2015 257 903

## Beschreibung

Die Erfindung betrifft eine Befestigungsanordnung einer Prothesenhand an einem Unterarmschaft mit einem an dem Unterarmschaft festlegbaren Aufnahmerahmen, an dem die Prothesenhand festgelegt ist, sowie ein Prothesensystem mit einem Unterarmschaft und einer über die Befestigungsanordnung daran festgelegten Prothesenhand. Die Erfindung betrifft insbesondere die Befestigungsanordnung für eine sogenannte Transcarpalhand, die bei einer Versorgung verwendet wird, bei der ein langer Unterarmstumpf erhalten werden konnte.

Prothesenhände, unter denen auch passive Prothesenhände, aktive Prothesenhände, reine kosmetische Prothesenhände oder auch Greifeinrichtungen wie sogenannte Hooks oder dergleichen verstanden werden, werden über einen Unterarmschaft an einem Prothesennutzer festgelegt. Der Unterarmschaft weist in der Regel eine formstabile Außenhülle auf, die mehr oder weniger exakt an die Kontur des Unterarms angepasst ist. Zwischen dem Unterarmstumpf und der Außenhülle kann ein Liner oder eine Polsterung angeordnet sein, um Volumenschwankungen des Stumpfes auszugleichen, ein Interface herzustellen und Druckkräfte abzupolstern. Über verschiedene weitere Befestigungseinrichtungen wie Unterdruckerzeuger oder Gurt und Manschetten kann der Unterarmschaft an dem Stumpf ggf. zusätzlich an dem Arm fixiert werden. An dem distalen Ende des Unterarmschaftes wird über eine Befestigungsanordnung, beispielsweise ein Bajonettsystem oder eine Verschraubung, die eigentliche Prothesenhand an dem Unterarmschaft befestigt. Die lösbare Befestigung der Prothesenhand an dem Unterarmschaft und der dadurch erreichte modulare Aufbau der gesamten Prothese ermöglicht es, dass die Prothesenhand als separate Komponente gefertigt werden kann. Lediglich der Unterarmschaft ist individuell an den jeweiligen Patienten angepasst. Darüber hinaus ist aufgrund der häufig mechanisch aufwendigen Bauweise und der Anordnung von Antrieben und gegebenenfalls Steuerungseinrichtungen und Energiespeichern innerhalb der Prothesenhand eine einstückige Ausgestaltung von Unterarmschaft und Prothesenhand nicht möglich. Die Unterbringung der mechanischen und elektronischen Komponenten innerhalb der Prothesenhand ist trotz der voranschreitenden Miniaturisierung schwierig, ebenso benötigen Energiespeicher, elektronische Steuerungen sowie Sensoren oder mechanische Kraftübertragungseinrichtungen Raum, der insbesondere bei Patienten mit einem niedrigen Amputationsniveau, also beispielsweise Amputationen im Bereich der Handwurzel, nicht oder nur unzureichend zur Verfügung steht. Dies hat zur Folge, dass die Prothesenhand über die natürliche Hand hinausragt, so dass eine ungleichmäßige Anmutung erzielt wird.

Aus der GB 2 498 788 A ist eine Anbindung einer Prothesenhand an einen Prothesenschaft bekannt, bei der ein Scheibenteil formschlüssig in einen Außenring einsetzbar ist. Das Scheibenteil weist Vorsprünge auf, die in korrespondierend ausgebildete Ausnehmungen in dem Ringteil angeordnet werden können. Nach Einsetzen des Scheibenteils in den Außenring kann das Scheibenteil verdreht werden. Die Befestigung einer Prothesenhand wird über eine durch eine Klemmung fixierte Verschraubung gewährleistet.

Die US 2011/0257765 A1 betrifft einen Prothesenarm, dessen Vielzahl an Segmenten untereinander verbindbar sind und mit einer Befestigungsvorrichtung verbunden werden können.

Aufgabe der vorliegenden Erfindung ist es, eine Befestigungsanordnung einer Prothesenhand an einem Unterarmschaft sowie eine Protheseneinrichtung mit Befestigungsanordnung, Prothesenhand und Unterarm schaft bereitzustellen, mit der eine verbesserte physiologische Anmutung auch bei Patienten mit einem niedrigen Amputationsniveau ermöglicht werden kann.

Erfindungsgemäß wird diese Aufgabe durch eine Befestigungsanordnung mit den Merkmalen des Hauptanspruches und eine Protheseneinrichtung mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Die erfindungsgemäße Befestigungsanordnung einer Prothesenhand an einem Unterarmschaft mit einem an dem Unterarmschaft festlegbaren Aufnahmerahmen, an dem die Prothesenhand festgelegt ist, sieht vor, dass in dem Aufnahmerahmen radial gerichtete Öffnungen ausgebildet sind, durch die Befestigungselemente hindurchragen, die formschlüssig mit einem proximalen Anschlussabschnitt der Prothesenhand in Eingriff stehen. Der Unterarmschaft umschließt einen Unterarmstumpf zumindest in einem distalen Bereich vollumfänglich, um eine stabile Aufnahme der Prothesenhand zu gewährleisten. Innerhalb des Unterarmschaftes oder an dem Unterarmschaft ist ein Aufnahmerahmen angeordnet, innerhalb dessen zumindest Teile einer Prothesenhand oder von Bauteilen einer Prothesenhand aufgenommen werden. Die Teile der Prothesenhand oder die Komponenten der Prothesenhand ragen in den Aufnahmerahmen hinein und werden von dem Aufnahmerahmen radial oder an ihren Außenseiten umgeben. Zur Befestigung der Prothesenhand an dem Aufnahmerahmen und damit auch an dem Unterarmschaft sind in dem Aufnahmerahmen radial gerichtete Öffnungen, insbesondere Bohrungen oder bereits bei dem Herstellverfahren des Aufnahmerahmens vorgesehene Ausnehmungen oder Durchbrüche vorhanden oder ausgebildet, durch die die Befestigungselemente hindurchragen. Diese Befestigungselemente ermöglichen es, formschlüssig mit einem proximalen Anschlussabschnitt der Prothesenhand in Eingriff zu treten, wenn die Prothesenhand an dem Unterarmschaft festgelegt wird. Der Anschlussabschnitt der Prothesenhand ist in dem montierten Zustand dem Unterarmschaft zugewandt und dient insbesondere der mechanischen Festlegung der Prothesenhand an dem Unterarmschaft. Die Befestigungselemente ermöglichen es, eine reversible Festlegung der Prothesenhand an dem Unterarmschaft durchzuführen, wobei sie radial von außen zugänglich sind, so dass nach dem Einführen des Anschlussabschnittes in den Aufnahmerahmen eine formschlüssige Verriegelung zwischen dem Anschlussabschnitt und dem Aufnahmerahmen stattfinden kann. Durch die radiale Orientierung der Befestigungselemente wird kein oder kaum Bauraum in der Längserstreckung des Unterarmschaftes benötigt, so dass die Prothesenhand sehr nahe an dem Unterarmschaft befestigbar ist, so dass sie nicht in Distalrichtung weit über das distale Ende des Unterarmschaftes hinausragt. Darüber hinaus ist durch die seitliche oder radiale Ausrichtung eine leichte Zugänglichkeit zu den Befestigungselementen gegeben, so dass die Prothesenhand leicht bei einem angelegten Prothesenschaft abgenommen werden kann, beispielsweise um die Prothesenhand zu reparieren oder Einstellungen daran vorzunehmen. Der Anschlussabschnitt ist lösbar an einem Grundkörper befestigt, wobei an dem Grundkörper weitere Komponenten der Prothesenhand befestigt sind.

Der Aufnahmerahmen und der Anschlussabschnitt können daran ausgebildete oder befestigte Verriegelungselemente aufweisen, die formschlüssig in Eingriff gebracht werden können, beispielsweise nach Art eines Bajonettverschlusses, bei dem ein Vorsprung innerhalb einer Führung verschoben und anschließend um einen festgelegten Winkelbereich verdreht wird, bis ein Anschlag erreicht wird. In dieser Stellung ist der Anschlussabschnitt formschlüssig mit dem Aufnahmerahmen verriegelt. Um ein ungewolltes Lösen der Verriegelung zu verhindern, werden Befestigungselemente an den dafür vorgesehenen Positionen radial eingeführt und blockieren eine Rückwärtsrotation in eine Entriegelungsstellung. Dies setzt voraus, dass eine Rotation des Anschlussabschnittes relativ zu dem Aufnahmerahmen möglich ist.

Eine physiologisch angenäherte Ausgestaltung des Aufnahmerahmens wird erreicht, wenn dieser eine nicht rotationssymmetrische Innenkontur aufweist, insbesondere eine ovale oder elliptische Innenkontur aufweist, die im Wesentlichen dem Querschnitt der Handwurzel entspricht. Durch die nicht rotationssymmetrische Innenkontur wird eine eindeutige Zuordnung des Anschlussabschnittes der Prothesenhand zu dem Aufnahmerahmen ermöglicht und eine Rotation um eine Achse in Längserstreckung des Unterarmschaftes verhindert. Dadurch kann eine besonders einfache Herstellung des Aufnahmerahmens mit einer glattwandigen Innenkontur erreicht werden. Darüber hinaus erfolgt eine gute Kraftübertragung über den gesamten Aufnahmerahmen aufgrund der nicht rotationssymmetrischen Innenkontur, wodurch die Stabilität und Haltbarkeit der Befestigungsanordnung erhöht wird. Aufgrund der nicht rotationssymmetrischen Innenkontur des Aufnahmerahmens kann bei vorbestimmten Positionen der Öffnungen oder Bohrungen eine präzise Ausrichtung der Befestigungselemente zu den Abschnitten des Anschlussabschnittes erfolgen, die mit den Befestigungselementen formschlüssig in Eingriff stehen oder in Eingriff gebracht werden sollen.

Der Aufnahmerahmen kann eine sich in Axialrichtung erstreckende, vorzugsweise umlaufende Wand aufweisen, von der sich ein Absatz radial nach außen erstreckt. Der sich in Radialrichtung erstreckende Absatz bildet bevorzugt den distalen Abschluss des Unterarmschaftes, so dass durch den Absatz eine Anlagefläche für die Prothesenhand bzw. für einen Anschlussabschnitt der Prothesenhand bereitgestellt wird. Durch die Ausgestaltung des Absatzes als distalen Abschluss werden insbesondere faserverstärkte Kunststoffe, die zur Herstellung eines Unterarmschaftes verwendet werden, mechanisch geschützt, wodurch die Haltbarkeit der gesamten Prothesenanordnung verbessert wird. Der Aufnahmerahmen kann den distalen Abschluss eines an dem distalen Ende offenen Unterarmschaftes ausbilden, so dass eine Durchtrittsöffnung innerhalb der Innenkontur des Aufnahmerahmens ausgebildet wird. Der Unterarmschaft ist dann rohrförmig ausgebildet, gegebenenfalls mit einer Verjüngung im distalen Endbereich, in dem der Aufnahmerahmen eine Öffnung innen und ggf. distal umgibt. Grundsätzlich ist es auch möglich, dass der Unterarmschaft distal geschlossen ist und der Aufnahmerahmen den distalen Endabschnitt umgibt. Der Aufnahmerahmen kann dann bevorzugt den Unterarmstumpf radial umgeben, um möglichst wenig Bauraum in Längserstreckung zu benötigen.

An der sich in Axialrichtung erstreckenden Wand des Aufnahmerahmens können radial abstehende Vorsprünge oder Hinterschnitte ausgebildet sein, mit denen es erleichtert wird, den Aufnahmerahmen an oder in dem Unterarmschaft festzulegen. Über die Vorsprünge oder Hinterschnitte kann die Festlegung des Unterarmschaftes erleichtert werden, insbesondere wenn der Aufnahmerahmen in dem Unterarmschaft einlaminiert wird. Die Vorsprünge und/oder Hinterschnitte können sich in Proximal-/ Distalrichtung und/oder umlaufend in Radialrichtung erstrecken, um einerseits eine Verdrehung um die Längserstreckung des Unterarmschaftes und andererseits eine Auszugsbewegung in Distalrichtung aus dem Unterarmschaft zu vermeiden.

Der Grundkörper kann beispielsweise einen motorischen Antrieb, eine Steuerung, Kraftübertragungselemente von einem Antrieb zu Prothesenfingern oder beweglichen Komponenten der Prothesenhand sowie Lagerungselemente oder Achslager zur Festlegung von beweglichen Komponenten aufweisen. Der Anschlussabschnitt ebenso wie der Grundkörper kann aus einem Metall, insbesondere Leichtmetall, gefertigt sein. Die lösbare Befestigung des Anschlussabschnittes an dem Grundkörper ermöglicht es, unterschiedliche Anschlussabschnitte an dem Grundkörper festzulegen, so dass Standardgrundkörper oder Standardprothesenhände mit unterschiedlichen Anschlussabschnitten versehen werden können, um eine individuelle Anpassung an Armstümpfe zu erreichen.

Eine Weiterbildung der Erfindung sieht vor, dass der Anschlussabschnitt verdrehbar und in der jeweiligen verdrehten Stellung fixierbar an dem Grundkörper festgelegt ist, um eine rotatorische Ausrichtung des Grundkörpers und damit der distalen Komponente der Prothesenhand an den Unterarmschaft zu erreichen. Auf diese Weise ist eine verbesserte Ausrichtung der Prothesenhand relativ zu dem Unterarmschaft und damit zu dem Unterarm des Patienten möglich. Fertigungsungenauigkeiten können ausgeglichen werden oder aber physiologische Besonderheiten kompensiert werden.

An dem Anschlussabschnitt kann zumindest ein Langloch, insbesondere ein teilkreisförmiges Langloch ausgebildet sein, das mit einem an dem Grundkörper befestigten Fixierelemente, das in dem Langloch aufgenommen ist, wechselwirkt und somit nach Erreichen der gewünschten Stellung fixiert oder geklemmt werden kann. Grundsätzlich ist es auch möglich, dass in dem Grundkörper ein Langloch ausgebildet ist, das in der Form der vorzunehmenden Bewegung relativ zu dem Anschlussabschnitt ausgeführt ist. Neben einer teilkreisförmigen Ausgestaltung des Langloches, über die eine rotatorische Orientierung erfolgt, kann auch eine andere Form des Langloches vorgesehen sein, um neben einer rotatorischen Veränderung auch eine translatorische Verlagerung vom Grundkörper zum Anschlussabschnitt zu ermöglichen. Grundsätzlich können auch rein geradlinige Langlöcher vorgesehen sein. Das Fixierelement ist insbesondere als Schraube ausgebildet, um eine einfache und reversible Fixierung des Anschlussabschnittes relativ zu dem Grundkörper durchführen zu können.

In einer Weiterbildung der Erfindung ist vorgesehen, dass der Anschlussabschnitt mit einer Zentralschraube an dem Grundkörper festgelegt ist, um eine grundsätzliche Positionierung des Grundkörpers an dem Anschlussabschnitt zu gewährleisten, bevor über eine Verdrehung und/oder Verschiebung und Fixierung der jeweiligen Position über die Fixierelemente oder das Fixierelement eine endgültige Orientierung des Anschlussabschnittes zu dem Grundkörper durchgeführt wird.

Der Anschlussabschnitt kann sich in Richtung auf den Unterarmschaft erstreckende Absätze oder eine korrespondierend orientierte, bevorzugt umlaufende Wand aufweisen. Die Absätze oder die Wand erstrecken sich somit in Proximalrichtung. Die Absätze sind korrespondierend zu den Öffnungen in dem Aufnahmerahmen positioniert und befinden sich in Überdeckung mit den Öffnungen, wenn der Anschlussabschnitt innerhalb des Aufnahmerahmens angeordnet ist. Dadurch ist es möglich, das Befestigungselement oder die Befestigungselemente in die Absätze einzuführen oder an den Absätzen eine entsprechende Fixierung vorzunehmen. Alternativ zu der Ausgestaltung mit einzelnen, zumindest zwei Absätzen kann eine Wand an dem Anschlussabschnitt angeordnet oder augebildet sein, in der zumindest ein Gewinde eingearbeitet oder angeordnet ist, das korrespondierend zu der Öffnung oder den Öffnungen in dem Aufnahmerahmen positioniert ist. Das zumindest eine Befestigungselement wird in das Gewinde eingeführt, um eine Fixierung zu ermöglichen. Dazu sind in einer Weiterbildung der Erfindung Gewinde in dem Anschlussabschnitt oder den Anschlussabschnitten eingearbeitet, die im vorgesehenen Montagezustand des Aufnahmerahmens zu dem Anschlussabschnitt korrespondierend zu den Öffnungen positioniert sind, so dass durch radiales Einführen von Schrauben durch die Öffnungen in die Gewinde in dem Absatz oder in den Absätzen oder der Wand der Aufnahmerahmen formschlüssig und reversibel in dem Anschlussabschnitt festgelegt wird.

Der Anschlussabschnitt kann an einer Grundplatte eines Grundkörpers befestigt sein, um so den Grundkörper modular aufbauen zu können. Über die Grundplatte erfolgt die Zuordnung des Anschlussabschnittes zu dem Grundkörper, ebenso ist es möglich, dass über die Grundplatte die übrigen Komponenten der Prothesenhand reversibel mit dem Anschlussabschnitt gekoppelt werden können. Dadurch ist es möglich, unterschiedliche Komponenten einer Prothesenhand oder unterschiedliche Modelle oder Größen einer Prothesenhand an einer Grundplatte festzulegen, um eine vorgegebene Schnittstelle zu einem Anschlussabschnitt bereitstellen zu können. Alternativ zu einer mehrteiligen Ausgestaltung des Grundkörpers aus Grundplatte und Träger kann der Grundkörper auch einteilig ausgebildet sein.

Vorteilhafterweise ist der Träger mit der Grundplatte formschlüssig gekoppelt, beispielsweise angeschraubt oder über eine Schwalbenschwanzführung mit einer zusätzlichen Fixierung daran befestigt. Der Grundkörper kann auch mit der Grundplatte formschlüssig gekoppelt, beispielsweise angeschraubt oder über eine Schwalbenschwanzführung mit einer zusätzlichen Fixierung daran befestigt sein.

Der Anschlussabschnitt kann eine Außenkontur aufweisen, die korrespondierend zu der Innenkontur des Aufnahmerahmens ausgebildet ist, um eine möglichst eindeutige Zuordnung des Anschlussabschnittes zu dem Aufnahmerahmen zu ermöglichen und darüber hinaus über eine möglichst gleichmäßige Anlage des Anschlussabschnittes an dem Aufnahmerahmen eine gleichmäßige Krafteinleitung zu bewirken.

Der Aufnahmerahmen ist bevorzugt in dem Unterarmschaft einlaminiert und kann über die Befestigungselemente zusätzlich an dem Unterarmschaft gesichert sein. Die Befestigungselemente sind bevorzugt als Schrauben oder Bolzen ausgebildet.

In dem Anschlussabschnitt und in dem Grundkörper kann zumindest eine Durchführung für Kabel oder andere Datenleitungen oder Kraftübertragungseinrichtungen von dem Unterarmschaft zu der Prothesenhand ausgebildet sein, um eine Aktivierung der Prothesenhand entweder mechanisch oder elektronisch zu ermöglichen.

Der Anschlussabschnitt weist bevorzugt einen inneren Freiraum auf, der von den Absätzen umgeben ist, so dass innerhalb der Absätze oder innerhalb desjenigen Bereiches, der von den Absätzen umgeben ist, weitere Komponenten der Prothesenhand angeordnet sein können, beispielsweise ein Energiespeicher, Steuerungskomponenten, Sensorik, Stecker oder Kabel.

Die Erfindung betrifft ebenfalls ein Prothesensystem oder eine Protheseneinrichtung mit einer Prothesenhand, die über eine wie oben beschriebene Befestigungsanordnung an einem Unterarmschaft festgelegt ist.

Nachfolgend werden Ausführungsbeispiele der Erfindung anhand der beigefügten Figuren näher erläutert. Es zeigen:
- Figur 1 -: eine Explosionsdarstellung einer Befestigungsanordnung einer Prothesenhand;
- Figur 2 -: eine teilmontierte Darstellung einer Prothesenhand mit einem Aufnahmerahmen in der Draufsicht;
- Figur 3 -: eine Seitenansicht einer Ausführungsform gemäß Figur 2;
- Figur 4 -: eine Draufsicht auf eine fertigmontierte Prothesenhand an einem Aufnahmerahmen;
- Figur 5 -: eine Seitenansicht gemäß Figur 4;
- Figur 6 -: eine Untenansicht in Neutralstellung;
- Figur 7 -: eine im Uhrzeigersinn verdrehte Prothesenhand in Untenansicht; sowie
- Figur 8 -: eine entgegen dem Uhrzeigersinn verdrehte Prothesenhand in Untenansicht.

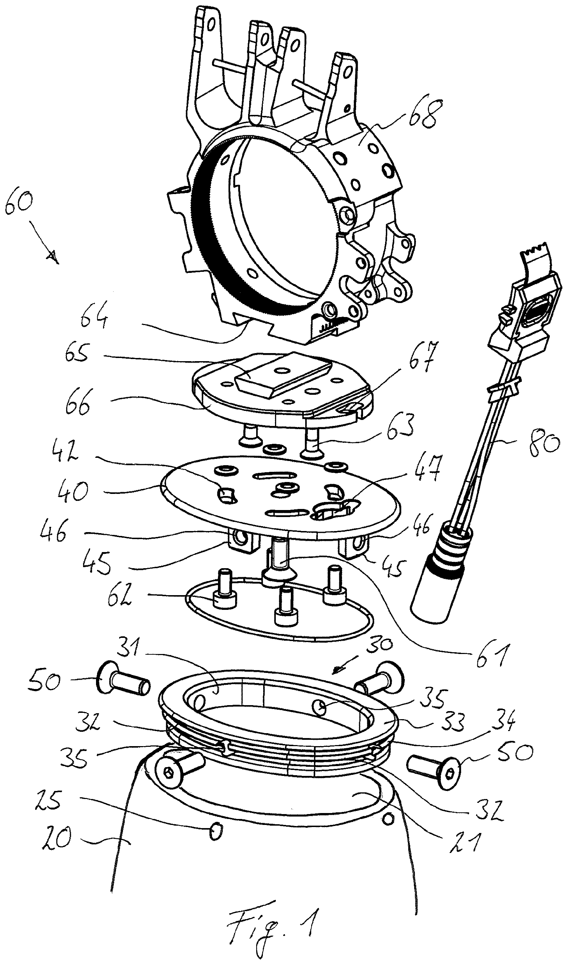

Figur 1 zeigt in einer Explosionsdarstellung die wesentlichen Komponenten einer Prothesenhand, die später näher erläutert wird, mit einem angedeuteten Unterarmschaft 20, der zur Aufnahme eines Aufnahmerahmens 30 ausgebildet ist. Der Unterarmschaft 20 ist in dem dargestellten Distalbereich mit einem geschlossenen Umfang ausgebildet und weist als distalen Endabschnitt eine Durchgangsöffnung 21 auf, innerhalb der ein Aufnahmerahmen 30 einsetzbar ist und eingesetzt wird. Der Aufnahmerahmen 30 weist eine ovale Außenkontur und eine ovale Innenkontur 31 auf und entspricht im Wesentlichen mit seiner Außenkontur der Innenkontur im Bereich der Öffnung 21 des Prothesenschaftes 20. Die Form ist einem Stumpfquerschnitt im Bereich der Handwurzel angenähert. Der Aufnahmerahmen 30 weist eine sich in Axialrichtung, also in Längserstreckung des Unterarmschaftes 20 erstreckende Wand 32 auf, von der sich als distaler Abschluss ein radial nach außen abstehender Absatz 33 erstreckt. Der Absatz 33 kann auf dem distalen Abschlussrand des Unterarmschaftes 20 aufliegen und somit den distalen Abschluss des Unterarmschaftes 20 bilden. Alternativ kann der Anschlussrahmen 30 auch in dem Prothesenschaft 20 so eingelassen werden, dass er bündig mit dem distalen Rand abschließt.

Innerhalb des Prothesenschaftes 20 sind Öffnungen oder Bohrungen 25 ausgebildet oder eingebracht, die durch die Wandung des Prothesenschaftes 20 hindurchgehen und es ermöglichen, dass Befestigungselemente 50 in Gestalt von Schrauben eingeschraubt oder hindurchgesteckt werden können. Innerhalb des Aufnahmerahmens 30 sind in der Wand 32 Bohrungen 35 oder Öffnungen 35 eingebracht oder ausgebildet, die korrespondierend zu den Bohrungen oder Öffnungen 25 in dem Prothesenschaft positioniert sind. Die Öffnungen 25 werden beispielsweise nach dem Einlaminieren des Anschlussrahmens 30 von innen nach außen durchstoßen, um die exakte Position der Öffnungen 25 zu übertragen. In den Bohrungen 35 können Gewinde ausgebildet oder angeordnet sein. Die Schrauben 50 können somit sowohl durch die Außenwand des Prothesenschaftes 20 als auch durch die Wand 32 des Aufnahmerahmens 30 hindurchragen, so dass sie im eingeschraubten oder eingesteckten Zustand in die Innenkontur 31 des Aufnahmerahmens 30 hineinragen. Grundsätzlich ist es auch möglich, dass die Befestigungselemente 50 oder Schrauben nicht durch den Prothesenschaft 20 hindurchgeführt werden, wenn der Aufnahmerahmen 30 distal über das Ende des Prothesenschaftes 20 hinausragt. Die Innenkontur 31 ist im dargestellten Ausführungsbeispiel nicht rotationssymmetrisch, sondern oval oder ellipsoid. Grundsätzlich besteht auch die Möglichkeit, die Innenkontur 30 rotationssymmetrisch auszugestalten oder eine polygonale Form der Innenkontur 31 zu wählen.

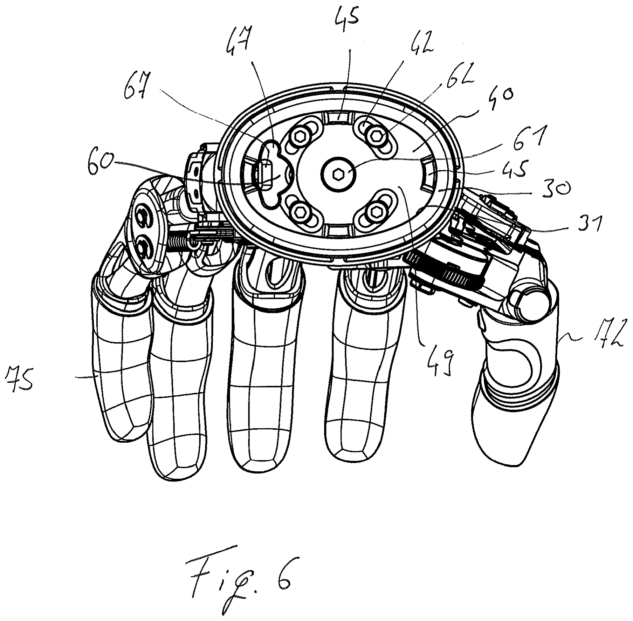

In der Figur 1 ist weiterhin ein Träger 68 einer Prothesenhand dargestellt. An dem Träger 68 können weitere Komponenten der Prothesenhand angeordnet sein, beispielsweise in der zentralen Ausnehmung ein motorischer Antrieb sowie an den äußeren Lagerstellen beweglich gelagerte Prothesenfinger bzw. ein Prothesendaumen. Der Träger 68 weist eine Schwalbenschwanzaufnahme 64 an seiner proximalen Unterseite auf, die eine formschlüssige Verriegelung mit einer korrespondierenden schwalbenschwanzartig ausgestalteten Führung 65 ermöglicht. Zentral innerhalb der Schwalbenschwanzführung 65, die an einer Grundplatte 66 angeordnet oder ausgebildet ist, ist eine Bohrung mit einem Gewinde angeordnet, in das eine zentral angeordnete Schraube 61 eingeschraubt werden kann. Die Schraube 61, die auch in den Figuren 6 bis 8 dargestellt ist, wird von der Proximalrichtung, also in der dargestellten Zeichnungsorientierung von unten, durch einen Anschlussabschnitt 40 hindurch in die Grundplatte 66 eingeschraubt, so dass die Grundplatte 66 mit dem Anschlussabschnitt 40, der als Platte ausgebildet ist und sich nach unten erstreckende Vorsprünge 45 aufweist, montiert werden kann. Zusätzlich sind zwei Schrauben 63 seitlich neben der Schwalbenschanzführung 65 durch die Grundplatte 66 hindurchgeführt und in nicht zu erkennende Schraublöcher innerhalb des Trägers 68 beiderseits der Schwalbenschwanzaufnahme 64 eingeschraubt, um eine formschlüssige und reversible Verbindung zwischen dem Träger 68 und der Grundplatte 66 bereitzustellen. An der Grundplatte 66 ist eine Durchführung 67 an einer Seite ausgebildet, durch die Kabel 80 während der Montage hindurchgeführt werden können, um eine Verbindung von elektrischen oder elektronischen Komponenten oder Energiespeichern im Bereich des Prothesenschaftes 20 zu den Sensoren oder elektrischen Verbrauchern innerhalb der Prothesenhand bereitzustellen. Die Durchführung 67 ist seitlich offen, so dass die Kabel 80 seitlich eingeführt und hindurchgeführt werden können. Die Grundplatte 66 ist ebenfalls oval geformt, wobei die Durchführung 67 in der Grundplatte 66 eine seitliche Öffnung an einem äußeren Scheitelpunkt aufweist.

Proximal zu der Grundplatte 66 ist der plattenförmige Anschlussabschnitt 40 mit den nach unten, also proximal abstehenden Vorsprüngen 45 angeordnet und an der Grundplatte 66 mit vier Schrauben 62, die die Fixierelemente für den Anschlussabschnitt 40 an der Grundplatte 66 bilden, fixiert. Die Fixierelemente 62 greifen in vier Gewindebohrungen innerhalb des Grundkörpers 66 ein und durchragen den plattenförmigen Anschlussabschnitt 40, in dem Langlöcher 42 angeordnet sind. Zum Befestigen und Orientieren des Anschlussabschnittes 40 an der Grundplatte 66 wird zunächst die Zentralschraube 61 durch die Zentralbohrung in dem ebenfalls ovalen Anschlussabschnitt 40 hindurchgeführt und mit dem Gewinde innerhalb der Grundplatte 66 in Eingriff gebracht. An der Grundplatte 66 kann der Träger 68 über die Schrauben 63 vorab festgelegt sein. Anschließend wird der Anschlussabschnitt 40 montiert, gegebenenfalls nachdem Kabel 80 durch eine in dem Anschlussabschnitt 40 ausgebildete Durchführung 67, die im montierten Zustand eine Überdeckung mit der Durchführung 47 in der Grundplatte 66 erlaubt, hindurchgeführt wurden. Wenn die Grundplatte 66 auf den Anschlussabschnitt 40 aufgeschraubt ist und die Kabel 80 durch die Durchführungen 47, 67 hindurchgeführt worden sind, kann die Grundplatte 66 relativ zu dem Anschlussabschnitt 40 um die Zentralschraube 61 verdreht werden, so dass eine rotatorische Ausrichtung zueinander möglich ist. Zur Endmontage werden die Fixierelemente 62 in Gestalt von Schrauben durch die Langlöcher 42 hindurchgeführt und mit der Grundplatte 66 in den darin ausgebildeten Gewinden verschraubt. Im dargestellten Ausführungsbeispiel sind die Langlöcher 42 als teilkreisförmige Langlöcher 42 ausgebildet, die auf einem gemeinsamen Umfang um die Zentralschraube 61 herum angeordnet sind, so dass eine Verdrehung der Grundplatte 66 weiterhin möglich ist, bis die Fixierelemente 62 eingeschraubt und angezogen werden, um die Grundplatte 66 mit dem Anschlussabschnitt 40 zu verklemmen.

Neben einer teilkreisförmigen Ausgestaltung der Langlöcher 42 um eine Zentralschraube 61 herum besteht auch die Möglichkeit, auf die Zentralschraube 61 zu verzichten und die Langlöcher 42 abweichend von einer teilkreisförmigen Kontur auszubilden, um neben einer Rotation auch eine Translation der Grundplatte 66 relativ zu dem Anschlussabschnitt 40 zu ermöglichen. Die Zentralschaube 61 kann auch in einem Langloch geführt sein, so dass auch bei Vorhandensein einer Zentralschraube 61 zur Vormontage sowohl einer Rotation als auch einer Verschiebung von Grundplatte 66 zu Anschlussabschnitt 40 möglich ist.

An der Unterseite des Anschlussabschnittes 40 sind im dargestellten Ausführungsbeispiel vier Vorsprünge 45 ausgebildet, die als Laschen oder Blöcke ausgebildet sind, in denen Gewinde 46 ausgebildet sind. Die Vorsprünge 45 sind von dem Außenumfang des plattenförmigen Anschlussabschnitts 40 etwas zurückgesetzt und korrespondieren mit der Innenkontur 31 des Aufnahmerahmens 30. Die Außenseiten der Vorsprünge 45 können im montierten Zustand an der Innenkontur 31 des Aufnahmerahmens 30 anliegen oder sind dicht dazu beabstandet. Die Vorsprünge 45 mit den Gewinden 46 sind so positioniert, dass sie im montierten Zustand mit den Öffnungen 35 innerhalb der Wand 33 fluchten, so dass die Befestigungselemente 50 durch die Öffnungen 35 in dem Aufnahmerahmen 30 hindurchgehen und in die Gewinde 46 eingreifen. Dadurch wird eine rotatorische und translatorische Festlegung des Anschlussabschnitts 40 an dem Aufnahmerahmen 30 und damit der Prothesenhand an dem Prothesenschaft 20 ermöglicht.

Der Aufnahmerahmen 30 kann in dem Prothesenschaft 20 einlaminiert sein. Die Öffnungen 25 können so groß ausgebildet sein, dass die Schraubenköpfe in dem Prothesenschaft 20 versenkt werden, gegebenenfalls können die Schraubenköpfe auch an der Wand 32 anliegen, so dass keine formschlüssige Verriegelung über die Schrauben zwischen dem Prothesenschaft 20 und dem Aufnahmerahmen 30 stattfindet. Die Vorsprünge 45 liegen bevorzugt an der Innenkontur 31 des Aufnahmerahmens 30 an, wobei innerhalb der Vorsprünge 45 ein Freiraum ausgebildet ist, in dem Kabel, elektronische Komponenten oder andere elektrische oder mechanische Komponenten angeordnet sein können, wobei diese um die Fixierelemente 62 herum oder zwischen diesen angeordnet sind.

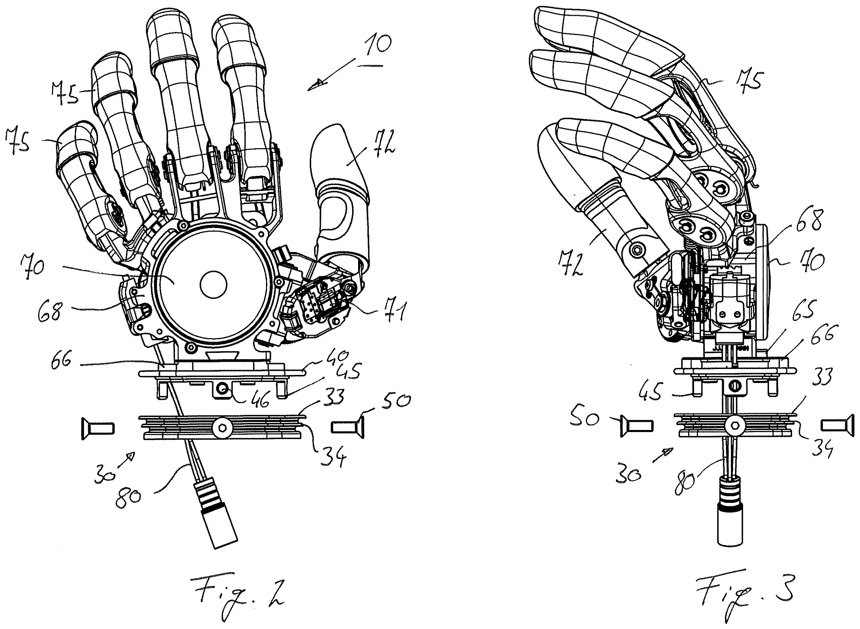

Figur 2 zeigt die Prothesenhand 10 mit der Befestigungsanordnung im teilmontierten Zustand, der Prothesenschaft 20 ist aus Übersichtlichkeitsgründen nicht dargestellt. In der Seitenansicht gemäß Figur 2, die eine Draufsicht auf den Handrücken zeigt, so wie in der Seitenansicht gemäß Figur 3, die eine Ansicht von der Handkantenaußenseite zeigt, ist gut zu erkennen, dass der Aufnahmerahmen 30 eine elliptische Form aufweist und an der Außenseite der Wand 32 ein Vorsprung 34 ausgebildet ist, mit dem eine verbesserte formschlüssige Anbindung innerhalb des nicht dargestellten Prothesenschaftes erreicht werden kann. Eine glattwandige Seitenwand 32 würde ein Einlaminieren und einen Rückhalt entgegen einer Auszugsbewegung in Distalrichtung erschweren, über den Vorsprung 34 und dem dadurch gebildeten Hinterschnitt distal und proximal dazu ist es möglich, eine formschlüssige Verriegelung von Prothesenschaft 20 und Aufnahmerahmen 30 zu erreichen.

Die Prothesenhand 10 mit den beweglich an dem Träger 68 gelagerten Prothesenfingern 75 und dem Prothesendaumen 72 mit dem eigenen Antrieb 71 sind in der Figur 2 zu erkennen, ebenso wie ein Zentralantrieb 70, der innerhalb der Ausnehmung in dem Träger 68 angeordnet ist. Über den Zentralantrieb 70 werden die motorisch angetriebenen Prothesenfinger 75 und der Prothesendaumen 72 um jeweils eine Schwenkachse aktuiert, der Prothesendaumen 72 kann über den Zusatzantrieb 71 eine weitere Verschwenkbewegung um eine zweite Schwenkachse ausführen. Der Träger 68 ist über die Schwalbenschwanzverbindung und die im Rahmen der Figur 1 beschriebenen Schrauben 63 und an der Grundplatte 66 festgelegt. Der Anschlussabschnitt 40 ist über die Fixierelemente 62 und die Zentralschraube 61 an der Grundplatte 66 lösbar befestigt. Von dem Anschlussabschnitt 40 ragen in Richtung auf den Aufnahmerahmen 30 insgesamt vier Vorsprünge 45 mit jeweils darin ausgebildeten Gewinden 46 ab, die korrespondierend zu den Öffnungen 35 innerhalb des Aufnahmerahmens 30 orientiert sind und somit eine formschlüssige, reversible Befestigung über die Befestigungselemente 50 in Gestalt von Schrauben ermöglichen. Die Kabel 80 sind durch die Durchführungen 47, 67 in der Grundplatte 66 und dem Anschlussabschnitt 40 hindurchgeführt und gegen durch den Innenraum zwischen den paarweise einander gegenüberliegenden Vorsprüngen 45 sowie den Freiraum innerhalb des geschlossenwandigen, ovalen, in der Mitte offenen Aufnahmerahmens 30 hindurch, so dass über eine Steckverbindung ein Anschluss zu einem Energiespeicher und elektronischen Steuerungseinrichtungen oder Sensoren bereitgestellt werden kann.

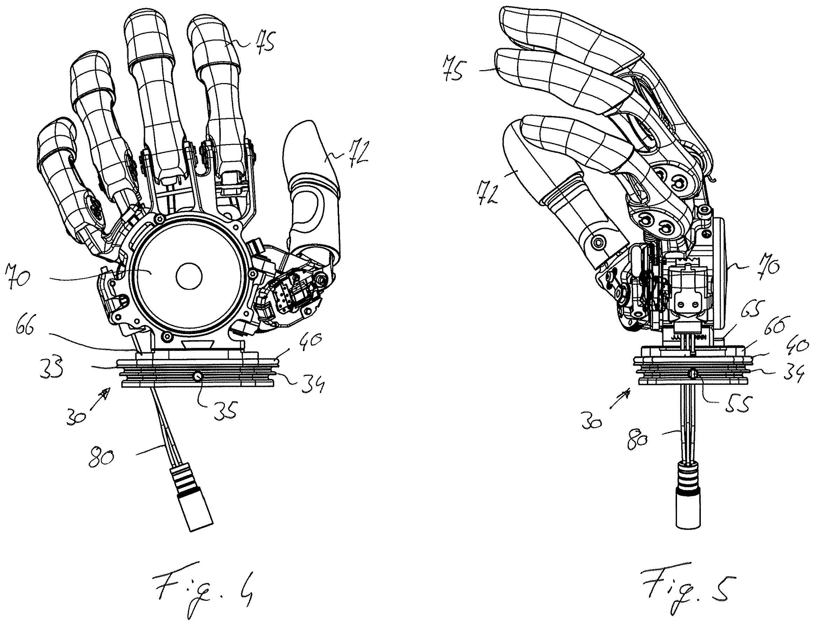

Die Figuren 4 und 5 zeigen die Prothesenhand 10 gemäß der Figuren 3 und 4 in einem ebenfalls teilmontierten Zustand, bei dem der Anschlussabschnitt 40 mit den Vorsprüngen in den Aufnahmerahmen 30 eingeführt ist. Die Befestigungselemente 50 in Gestalt von Schrauben sind nicht gezeigt, die Öffnungen 35 in Gestalt von Bohrungen innerhalb des Aufnahmerahmens 30 fluchten mit den Gewinden 46 der Vorsprünge 45, so dass nach dem Einführen und Einschrauben der Befestigungselemente 50 eine formschlüssige Verriegelung zwischen dem Aufnahmerahmen und dem Anschlussabschnitt 40 und damit auch zwischen der Prothesenhand 10 und dem nicht dargestellten Prothesenschaft bereitgestellt werden kann.

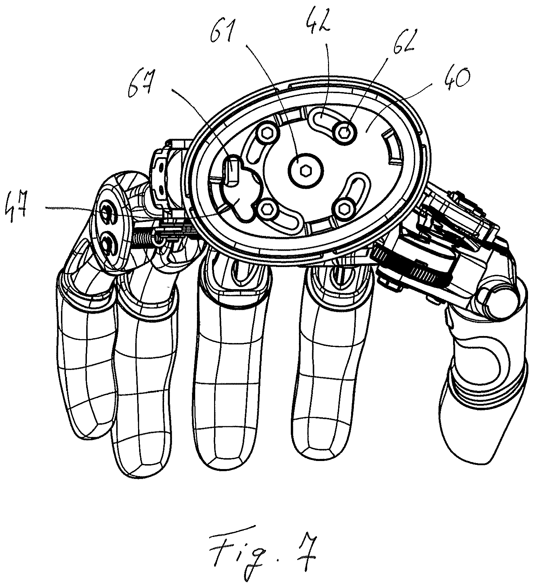

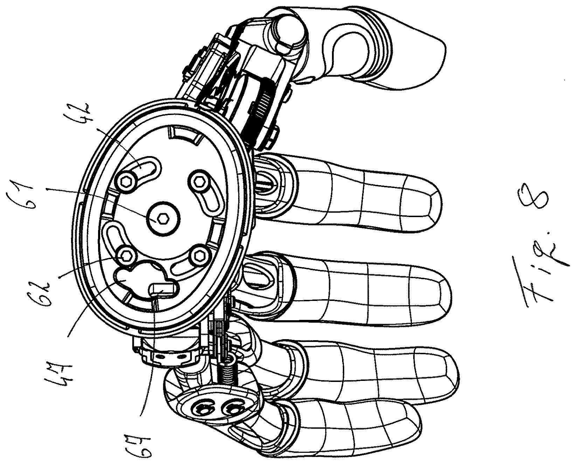

Figur 6 zeigt den teilmontierten Zustand gemäß der Figuren 4 und 5 in einer Untenansicht, in der das proximale Ende der Prothesenhand zu erkennen ist. Die Zentralschraube 61 ist im Zentrum des ellipsenförmigen Aufnahmerahmens 30 zu erkennen, der Anschlussabschnitt 40 mit den Vorsprüngen 45 ist innerhalb der Innenkontur 31 des Aufnahmerahmens 30 eingesetzt, die Schrauben sind noch nicht eingeschraubt. Die Fixierelemente 62 sind in den Langlöchern 42 angeordnet, ragen durch diese hindurch und sind in der Grundplatte 60 in die dafür vorgesehenen Gewinde eingeschraubt. Sowohl die Durchführung 47 in dem Anschlussabschnitt 40 als auch die Durchführung 67 in der Grundplatte 60 sind zu erkennen, ebenso ist zu erkennen, dass die Durchführungen 47, 67 einander überdecken. Die Fixierelemente 62 sind mittig innerhalb der teilkreisförmigen Langlöcher 42 angeordnet. Innerhalb des durch die Vorsprünge 45 umgebenen Raumes sind an der Unterseite des Anschlussabschnittes 40 keine Bauteile oder Strukturelemente ausgebildet, so dass die Vorsprünge 45 zusammen mit dem plattenförmigen Anschlussabschnitt 40 einen Freiraum 49 ausbilden oder definieren, in dem weitere Komponenten der Prothesenhand, Befestigungsmittel, Kabel, Steuerungen, Sensoren oder andere Bauteile angeordnet werden können. Der Freiraum 49 kann auch bei einer Ausgestaltung mit einer Wand oder einer proximal abstehenden Ringwand ausgebildet werden.
Zur rotatorischen Ausrichtung der Prothesenhand 10 relativ zu dem Unterarmschaft 20 kann die Prothesenhand 10 zusammen mit der Grundplatte 66 relativ zu dem Anschlussabschnitt 40 verdreht werden. Die Figur 7 zeigt die Ansicht gemäß Figur 6 in einer ersten, maximal in Uhrzeigerrichtung verdrehten Prothesenhand, bei der die Fixierelemente 62 oder Schrauben 62 jeweils an das Ende der Langlöcher 42 anschlagen. Durch die vergrößerte Ausgestaltung der Durchführung 47 im Vergleich zu der Durchführung 67 verbleibt immer noch eine Überdeckung der Durchführungen 47, 67, so dass die nicht dargestellten Kabel weder geknickt noch eingeklemmt werden. Figur 8 zeigt die entgegengesetzt verdrehte Maximalposition, bei der die Fixierelemente 62 an den gegenüberliegenden Enden der Langlöcher 42 anschlagen, auch hier ergibt sich eine Überdeckung der Durchführungen 47, 67 in dem Anschlussabschnitt 40 und der Grundplatte 66 für eine kollisionsfreie Durchführung der Kabel.

Durch den modularen Aufbau der Prothesenhand mit dem Träger 68 und der Grundplatte 66 kann eine auswechselbare, formschlüssige Verriegelung und Verbindung mit der Befestigungsanordnung und zur Befestigung an einen Prothesenschaft erreicht werden. Eine Befestigungsanordnung mit der Grundplatte 66, dem Anschlussabschnitt 40 sowie dem Aufnahmerahmen 30 kann jeweils individuell angepasst werden. So können unterschiedlich große Aufnahmerahmen 30 mit entsprechend ausgebildeten und angepassten Anschlussabschnitten 40 kombiniert werden, um eine Anpassung an unterschiedlich große Amputationsstümpfe oder Unterarmstümpfe verwirklichen zu können. Eine Zugänglichkeit zu den Fixierelementen 62 ist auch nach der Montage durch den Prothesenschaft 20 grundsätzlich gegeben, so dass für eine Feinjustierung lediglich die Zentralschraube 61 sowie die Fixierelemente 62 gelöst, die Grundplatte 66 relativ zu dem Aufnahmeabschnitt 40 verdreht und anschließend die Schrauben 61, 62 wieder angezogen werden müssen, um eine gewünschte Position zu fixieren.

## Patentansprüche

1. Befestigungsanordnung einer Prothesenhand (10) an einem Unterarmschaft (20) mit einem an dem Unterarmschaft (20) festlegbaren Aufnahmerahmen (30), an dem die Prothesenhand (10) festgelegt ist, **dadurch gekennzeichnet, dass** in dem Aufnahmerahmen (30) radial gerichtete Öffnungen (35) ausgebildet sind, durch die Befestigungselemente (50) hindurchragen, die formschlüssig mit einem proximalen Anschlussabschnitt (40) der Prothesenhand (10) in Eingriff stehen, wobei der Anschlussabschnitt (40) lösbar an einem Grundkörper (60) befestigt ist.

2. Befestigungsanordnung nach Anspruch 1, **dadurch gekennzeichnet, dass** der Aufnahmerahmen (30) eine nicht rotationssymmetrische Innenkontur (31) aufweist.

3. Befestigungsanordnung nach Anspruch 1 oder 2, **dadurch gekennzeichnet, dass** der Aufnahmerahmen (30) eine sich in Axialrichtung erstreckende Wand (32) und einen sich davon radial erstreckenden Absatz (33) aufweist.

4. Befestigungsanordnung nach Anspruch 3, **dadurch gekennzeichnet, dass** an der Wand (32) radial abstehende Vorsprünge (34) oder Hinterschnitte ausgebildet sind.

5. Befestigungsanordnung nach Anspruch 3 oder 4, **dadurch gekennzeichnet, dass** der Absatz (33) den distalen Abschluss des Unterarmschaftes (20) bildet.

6. Befestigungsanordnung nach einem der voranstehenden Ansprüche, **dadurch gekennzeichnet, dass** der Anschlussabschnitt (40) verdrehbar und fixierbar an dem Grundkörper (60) festgelegt ist.

7. Befestigungsanordnung nach einem der voranstehenden Ansprüche, **dadurch gekennzeichnet, dass** an dem Anschlussabschnitt (40) zumindest ein teilkreisförmiges Langloch (42) ausgebildet und an dem Grundkörper (60) ein in dem Langloch (42) aufgenommenes Fixierelement (62) (Schraube) befestigt ist.

8. Befestigungsanordnung nach einem der voranstehenden Ansprüche, **dadurch gekennzeichnet, dass** der Anschlussabschnitt (40) mit einer Zentralschraube (61) an dem Grundkörper (60) festgelegt ist.

9. Befestigungsanordnung nach einem der voranstehenden Ansprüche, **dadurch gekennzeichnet, dass** der Anschlussabschnitt (40) eine sich in Richtung auf den Unterarmschaft (20) erstreckende Wand oder Absätze (45), die korrespondierend zu den Öffnungen (35) in dem Aufnahmerahmen (30) positioniert sind, aufweist.

10. Befestigungsanordnung nach einem der voranstehenden Ansprüche, **dadurch gekennzeichnet, dass** in dem Anschlussabschnitt (40) Gewinde (46) eingearbeitet sind, die korrespondierend zu den Öffnungen (35) positioniert sind.

11. Befestigungsanordnung nach einem der voranstehenden Ansprüche, **dadurch gekennzeichnet, dass** der Grundkörper (60) mehrteilig ausgebildet ist und einen Träger (68) und einer daran befestigte Grundplatte (66) aufweist.

12. Befestigungsanordnung nach einem der voranstehenden Ansprüche, **dadurch gekennzeichnet, dass** der Grundkörper (60) mit der Grundplatte (66) formschlüssig gekoppelt ist.

13. Befestigungsanordnung nach einem der voranstehenden Ansprüche, **dadurch gekennzeichnet, dass** der Anschlussabschnitt (40) an der Grundplatte (66) lösbar befestigt ist.

14. Befestigungsanordnung nach einem der voranstehenden Ansprüche, **dadurch gekennzeichnet, dass** der Anschlussabschnitt (40) eine Außenkontur aufweist, die korrespondierend zu der Innenkontur des Aufnahmerahmens (30) ausgebildet ist.

15. Befestigungsanordnung nach einem der voranstehenden Ansprüche, **dadurch gekennzeichnet, dass** der Aufnahmerahmen (30) in dem Unterarmschaft (20) einlaminiert ist.

16. Befestigungsanordnung nach einem der voranstehenden Ansprüche, **dadurch gekennzeichnet, dass** in dem Anschlussabschnitt (40) und dem Grundkörper (60) zumindest eine Durchführung (47, 67) für Kabel von dem Unterarmschaft (20) zu der Prothesenhand (10) ausgebildet sind.

17. Befestigungsanordnung nach einem der voranstehenden Ansprüche, **dadurch gekennzeichnet, dass** der Anschlussabschnitt (40) einen inneren Freiraum (49) aufweist.

18. Prothesensystem mit einem Unterarmschaft (20), einer Prothesenhand (10) und einer Befestigungsanordnung nach einem der voranstehenden Ansprüche.

## Claims

1. A securing arrangement of a prosthetic hand (10) on a forearm socket (20) with a receiving frame (30) that can be fixed to the forearm socket (20), the prosthetic hand (10) being fixed to said receiving frame, **characterized in that** the receiving frame (30) be designed to feature radially-directed openings (35), through which the securing elements (50) protrude, wherein said securing elements positively engage with a proximal connection section (40) of the prosthetic hand (10), wherein the connection section (40) is fixed to a base body (60) such that it can be detached.

2. The securing arrangement according to claim 1, **characterized in that** the receiving frame (30) features an inner contour (31) that is not rotationally symmetrical.

3. The securing arrangement according to claim 1 or 2, **characterized in that** the receiving frame (30) comprises a wall (32) that extends in the axial direction and a ledge (33) that extends radially from said wall.

4. The securing arrangement according to claim 3, **characterized in that** radially protruding projections (34) or undercuts are configured on the wall (32).

5. The securing arrangement according to claim 3 or 4, **characterized in that** the ledge (33) forms the distal termination of the forearm socket (20).

6. The securing arrangement according to one of the above claims, **characterized in that** the connection section (40) be attached such that it can be twisted and fixed to the to the base body (60).

7. The securing arrangement according to one of the above claims, **characterized in that** the connection section (40) comprises at least one elongated hole (42) and that a fixing element (62) (screw) accommodated in the elongated hole (42) is fixed to the base body (60).

8. The securing arrangement according to one of the above claims, **characterized in that** the connection section (40) be attached to the base body (60) using a central screw (61).

9. The securing arrangement according to one of the above claims, **characterized in that** the connection section (40) features a wall or ledges (45) that extend towards the forearm socket (20), which are positioned correspondingly to the openings (35) in the receiving frame (30).

10. The securing arrangement according to one of the above claims, **characterized in that** threads (46) are incorporated in the connection section (40) which are positioned correspondingly to the openings (35).

11. The securing arrangement according to one of the above claims, **characterized in that** the base body (60) is designed to be multi-piece and comprises a support (68) and base plate (66) fixed to said support.

12. The securing arrangement according to one of the above claims, **characterized in that** the base body (60) is positively coupled with the base plate (66).

13. The securing arrangement according to one of the above claims, **characterized in that** the connection section (40) is fixed to the base plate (66) such that it can be detached.

14. The securing arrangement according to one of the above claims, **characterized in that** the connection section (40) features an outer contour that is designed correspondingly to the inner contour of the receiving frame (30).

15. The securing arrangement according to one of the above claims, **characterized in that** the receiving frame (30) is laminated in the forearm socket (20).

16. The securing arrangement according to one of the above claims, **characterized in that** at least one passage (47, 67) for cables from the forearm socket (20) to the prosthetic hand (10) is configured in the connection section (40) and in the base body (60).

17. The securing arrangement according to one of the above claims, **characterized in that** the connection section (40) features an inner free space (49).

18. A prosthesis system with a forearm socket (20), a prosthetic hand (10) and a securing arrangement according to one of the above claims.

## Revendications

1. Ensemble de fixation d'une main prothétique (10) à une emboîture d'avant-bras (20), comportant un cadre de réception (30) qui peut être immobilisé sur l'emboîture d'avant-bras (20) et auquel la main prothétique (10) est immobilisée,
**caractérisé en ce que** des ouvertures (35) orientées radialement sont ménagées dans le cadre de réception (30), qui sont traversées par des éléments de fixation (50) qui sont en engagement par coopération de forme avec une portion de raccordement proximale (40) de la main prothétique (10), la portion de raccordement (40) étant fixée de façon amovible à un corps de base (60).

2. Ensemble de fixation selon la revendication 1,
**caractérisé en ce que** le cadre de réception (30) présente un contour intérieur (31) qui n'est pas à symétrique de révolution.

3. Ensemble de fixation selon la revendication 1 ou 2,
**caractérisé en ce que** le cadre de réception (30) présente une paroi (32) s'étendant dans la direction axiale et un talon (33) s'étendant radialement à partir de celle-ci.

4. Ensemble de fixation selon la revendication 3,
**caractérisé en ce que** des saillies (34) en dépassement radial ou des contre-dépouilles sont formées sur la paroi (32).

5. Ensemble de fixation selon la revendication 3 ou 4,
**caractérisé en ce que** le talon (33) forme la terminaison distale de l'emboîture d'avant-bras (20).

6. Ensemble de fixation selon l'une des revendications précédentes,
**caractérisé en ce que** la portion de raccordement (40) est immobilisée sur le corps de base (60) de manière à pouvoir tourner et d'être fixée.

7. Ensemble de fixation selon l'une des revendications précédentes,
**caractérisé en ce qu'**au moins un trou oblong (42) en forme de cercle partiel est ménagé au niveau de la portion de raccordement (40), et un élément de fixation (62) (vis) logé dans le trou oblong (42) est fixé au corps de base (60).

8. Ensemble de fixation selon l'une des revendications précédentes,
**caractérisé en ce que** la portion de raccordement (40) est immobilisée sur le corps de base (60) par une vis centrale (61).

9. Ensemble de fixation selon l'une des revendications précédentes,
**caractérisé en ce que** la portion de raccordement (40) présente une paroi s'étendant en direction de l'emboîture d'avant-bras (20) ou présente des talons (45) qui sont positionnés de manière à correspondre aux ouvertures (35) dans le cadre de réception (30).

10. Ensemble de fixation selon l'une des revendications précédentes,
**caractérisé en ce que** des pas de vis (46) sont taillés dans la portion de raccordement (40), qui sont positionnés de manière à correspondre aux ouvertures (35).

11. Ensemble de fixation selon l'une des revendications précédentes,
**caractérisé en ce que** le corps de base (60) est réalisé en plusieurs pièces et comporte un support (68) et une plaque de base (66) fixée à ce dernier.

12. Ensemble de fixation selon l'une des revendications précédentes,
**caractérisé en ce que** le corps de base (60) est couplé par coopération de forme avec la plaque de base (66).

13. Ensemble de fixation selon l'une des revendications précédentes,
**caractérisé en ce que** la portion de raccordement (40) est fixée de manière amovible à la plaque de base (66).

14. Ensemble de fixation selon l'une des revendications précédentes,
**caractérisé en ce que** la portion de raccordement (40) présente un contour extérieur qui est réalisé de manière à correspondre au contour intérieur du cadre de réception (30).

15. Ensemble de fixation selon l'une des revendications précédentes,
**caractérisé en ce que** le cadre de réception (30) est intégré par stratification dans l'emboîture d'avant-bras (20).

16. Ensemble de fixation selon l'une des revendications précédentes,
**caractérisé en ce qu'**au moins un passage respectif (47, 67) pour des câbles allant de l'emboîture d'avant-bras (20) à la main prothétique (10) est ménagé dans la portion de raccordement (40) et dans le corps de base (60).

17. Ensemble de fixation selon l'une des revendications précédentes,
**caractérisé en ce que** la portion de raccordement (40) présente un espace intérieur libre (49).

18. Système de prothèse comportant une emboîture d'avant-bras (20), une main prothétique (10) et un ensemble de fixation selon l'une des revendications précédentes.
